# EUROPEAN PATENT SPECIFICATION

(11) **EP 4 355 406 B1**
(45) Date of publication and mention of the grant of the patent: **26.08.2026**
(21) Application number: 22809994.1
(22) Date of filing: 18.05.2022
(51) Int. Cl.: A61M 25/06

(54) **SYSTEMS FOR ACCESSING SMALL ARTERIES FOR CONVEYING CATHETERS TO TARGET VESSELS**
SYSTEME FÜR DEN ZUGANG ZU KLEINEN ARTERIEN ZUM TRANSPORT VON KATHETERN ZU ZIELGEFÄSSEN
SYSTÈMES POUR ACCÉDER À DE PETITES ARTÈRES POUR ACHEMINER DES CATHÉTERS VERS DES VAISSEAUX CIBLES

(30) Priority: 26.05.2021 US 202163193255 P
(43) Date of publication of application: 24.04.2024
(73) Proprietor: MG Stroke Analytics Inc., Calgary, Alberta T3H 5V4 (CA)
(72) Inventor: GOYAL, Mayank, Calgary, Alberta T3H 5V4 (CA); ARGUELLO, Edward, Pembroke Pines, Florida 33332 (US)
(74) Representative: Murgitroyd & Company
(86) International application number: PCT/CA2022/050787
(87) International publication number: WO 2022/246544

(56) References cited:
- EP-A1- 0 450 221
- WO-A1-2005/061034
- WO-A1-2006/087512
- WO-A1-2021/113962
- WO-A1-2021/113962
- DE-A1- 102013 008 316
- DE-U1- 202010 002 803
- US-A1- 2008 142 005
- US-A1- 2008 177 249
- US-A1- 2011 152 760
- US-A1- 2015 011 833
- US-A1- 2015 265 802
- US-A1- 2019 054 272
- US-A1- 2020 246 586

## Description

### FIELD

Systems and methods for accessing small arteries for conveying catheters to target vessels such as brain vessels are described including systems enabling a catheter to be introduced directly through a vessel opening without an external sheath wherein a distal tip of the catheter is protected by a protective cover. Methods of introducing catheters into vessels and kits are also described.

### BACKGROUND

Neuro-intervention (NI) procedures utilizing catheter systems to gain access to the cerebral arteries for the treatment of ischemic stroke are varied in terms of approach and the catheter systems utilized. Similarly, other intervention procedures to access other target vessels including the heart or other target areas through the vasculature utilize a range of catheter systems.

In many cases, and in particular NI and cardiac-intervention (CI) procedures, access to the vasculature is obtained through the femoral artery, mainly due to its size and its proximity to the skin. While the femoral artery is an advantageous access point, there are downsides to its use when NI and CI procedures are conducted through this location including longer recovery times within a treatment center before discharge with a commensurate increase in costs. For example, when a procedure is conducted through the femoral artery, a patient must typically be kept in a treatment center for a longer period of time due to the need for more time for the access wound to heal before allowing the patient to walk that requires additional resources to manage the patient during this time. In addition, particularly for obese patients, access to femoral artery can be challenging.

In comparison, if an NI or CI procedure is conducted through a radial artery, the patient can be discharged more quickly as the healing of the access wound does not prevent the patient becoming ambulatory almost immediately following the procedure. Hence, in an effort to save hospital and other treatment costs, there is a preference, when appropriate to conduct procedures via the arm arteries.

However, the radial/brachial arteries are smaller and thus generally present limitations and complications for certain procedures. Specifically, there is an upper limit on the size of catheters that be introduced into the arm arteries using conventional artery access equipment.

Patent publication US 2015/0265802 A1 discloses a catheter assembly.

### SUMMARY

The aforementioned objectives are achieved according to the invention by means of a system for introducing a catheter into a vasculature through a vessel opening according to claim 1, and equally by means of a kit according to claim 14. Preferred embodiments are defined in the dependent claims.

In accordance with one embodiment of the invention, a system for introducing a catheter into the vasculature through a vessel opening (VO) is described, the system including: a catheter having an internal diameter and external diameter; an internal guide sized for telescopic movement within the catheter, the internal guide for supporting the catheter and protecting a distal tip of the catheter as the catheter is introduced through a VO, the internal guide having: a tapered distal tip for introducing the system through the VO; and, a protective cover connected to the tapered distal tip extending proximally for engagement over the distal tip of the catheter, the protective cover moveable between an engaged position over the catheter and a disengaged position; wherein selective movement of the internal guide relative to the catheter causes the protective cover to move from the engaged position to the disengaged position and when in the disengaged position allows the internal support and protective cover to be proximally withdrawn through the catheter, wherein the protective cover is a sheath and the sheath inverts to the disengaged position during withdrawal of the internal support and where the sheath has at least one slit extending along a longitudinal length of the sheath, the slit for relieving friction between the catheter and the internal support.

In various preferred embodiments:
- the protective cover is a plurality of inwardly biased arms and the internal guide includes corresponding recesses for receiving the arms in a compressed position.
- the protective cover is an elastic sheath circumferentially covering the distal tip of the catheter in the engaged position.
- the elastic sheath seats against the internal guide in the disengaged position.
- the elastic sheath inverts to the disengaged position during withdrawal of the internal guide.
- the catheter has a catheter end stop and the internal guide has an internal guide end stop and wherein when the protective cover is in the engaged position, movement of the internal guide end stop towards the catheter end stop causes the protective cover to move to the disengaged position.
- the catheter is an aspiration catheter having a soft distal tip region and a length sufficient to extend from a radial artery VO to the cerebral vessels for the treatment of ischemic stroke.
- the aspiration catheter is 5-8F.
- the aspiration catheter is 6-8F.
- the internal guide is hollow, and the tapered distal tip includes a through bore allowing the internal guide to ride over a wire.
- the at least one slit extends to a proximal edge of the sheath.
- the at least one slit includes a zone of weakness capable of opening during inversion.
- the at least one slit does not extend across a proximal edge of the sheath.
- the at least one slit is a longitudinal slot.
- the sheath includes one or more voids to reduce the total volume of the sheath and facilitate sheath inversion during withdrawal of the internal guide.
- the protective cover overlaps the catheter by 1-5 cm when in the engaged position.
- the protective cover is connected to the tapered distal tip at a position where the outer diameter of the tapered distal tip is less than the maximum outer diameter of the internal guide.
- the system includes a band surrounding the distal end of the sheath for holding the sheath on the catheter prior to use.
- the protective cover includes a shaped band for applying a biasing force over the catheter to hold the protective cover in an engaged position on the catheter.
- the shaped band is a thermo material and wherein exposure to a body temperature relaxes the biasing force.
- the system includes an expandable ring, the expandable ring having an internal diameter expandable between an external diameter of the internal support and an external diameter of the catheter, the expandable ring having a distal edge having a thickness to be placed under a proximal edge of the elastic sheath during assembly of the elastic sheath on the catheter.
- the expandable ring is openable.

In another embodiment of the invention, a kit is described including: an internal support within sterilized packaging, the internal guide sized for telescopic movement within a catheter, the internal guide for supporting the catheter and protecting a distal tip of the catheter as the catheter is introduced through a VO, the internal guide having: a tapered distal tip for introducing the system through the VO; and, a protective cover connected to the tapered distal tip extending proximally for engagement over the distal tip of the catheter, the protective cover moveable between an engaged position over the catheter and a disengaged position; wherein selective movement of the internal guide relative to the catheter causes the protective cover to move from the engaged position to the disengaged position and when in the disengaged position allows the internal support and protective cover to be proximally withdrawn through the catheter, wherein the protective cover is a sheath and the sheath inverts to the disengaged position during withdrawal of the internal support and where the sheath has at least one slit extending along a longitudinal length of the sheath, the slit for relieving friction between the catheter and the internal support.

In various preferred embodiments:
- the kit includes the catheter and the internal support and catheter are packaged within the same sterilized packaging.
- the kit includes the catheter and the catheter and the internal support are in separate packages including a catheter package and an internal support package.
- the kit includes an expandable ring within the internal support package, the expandable ring having an internal diameter expandable between an external diameter of the internal support and an external diameter of the catheter, the expandable ring having a distal edge having a thickness to be placed under a proximal edge of the protective cover during assembly of the protective cover on the catheter.

In another aspect not forming part of the invention as claimed, a method of introducing a catheter into a vessel through a vessel opening VO is described comprising the steps of: puncturing a vessel with a hollow needle to form a VO; introducing a wire through the hollow needle; withdrawing the needle over the wire; introducing an arterial access assembly of an internal guide having a tapered proximal tip and a catheter supported over the internal guide, the catheter having a distal tip operatively engaged with a protective cover configured to the internal guide; advancing the assembly away from the VO; advancing the internal guide proximally relative to the catheter to disengage the protective cover from the distal tip of the catheter; and, withdrawing the internal guide and protective cover through the catheter.

### BRIEF DESCRIPTION OF THE DRAWINGS

Various objects, features and advantages will be apparent from the following description of particular embodiments, as illustrated in the accompanying drawings. The drawings are not necessarily to scale, emphasis instead being placed upon illustrating the principles of various embodiments. Similar reference numerals indicate similar components.
**Figures 1A-1G** are schematic diagrams showing equipment for conducting a typical procedure for gaining access to an artery in accordance with the prior art. Typical steps include needle puncture (Figure 1A), wire insertion (Figure 1B), needle removal (Figure 1C), arterial access system insertion (Figure 1D) and internal guide removal (Figure 1G). Figures 1E and 1F show an internal guide and external sheath respectively.
**Figures 2A-2C** are schematic diagrams of an arterial access system (AAS) having a distal tip protection system (DTPS) and a method (method not claimed) of gaining arterial access in accordance with one embodiment.
**Figures 3A-3D** are schematic diagrams of an arterial access system (AAS) having a distal tip protection system (DTPS) and a method (method not claimed) of gaining arterial access in accordance with another embodiment.
**Figures 3E-3I** are sketches of a number of representative and alternate sheaths in accordance with various embodiments in which the sheath has at least one longitudinal slit.
**Figures 3J and 3K** are schematic diagrams showing inversion of an "umbrella" DTPS system in accordance with two embodiments.
**Figures 3L-3O** are cross-sectional drawings showing the steps of inverting an umbrella/sheath.
**Figure 3P** is a sketch showing an assembly with a removable band that can be removed as the assembly is being inserted into a vessel.
**Figure 3Q** is a sketch of a removable band having two pull tabs allowing the band to be removed from an assembly.
**Figures 3R-3T** are sketches of a removable band tearing away upon insertion into a vessel.
**Figure 3T1**is a sketch showing assembly of a removable over an umbrella and AC.
**Figure 3U and 3V** are sketches of an umbrella having a proximal edge overlap and memory material proximal edge.
**Figures 4A-4F** are schematic diagrams showing a method (not claimed) of assembling an arterial access system (AAS) having a distal tip protection system (DTPS) on an aspiration catheter in accordance with one embodiment. Figure 4F shows an assembly ring in accordance with one embodiment.
**Figure 5** is a flow chart showing the steps for assembling an AAS at a treatment center in accordance with one embodiment.

### DETAILED DESCRIPTION

### Rationale

The inventors who have experience in the treatment of acute ischemic stroke recognized that a problem exists in introducing larger diameter aspiration catheters into smaller arteries, such as the radial artery, utilizing current artery access equipment. The systems and methods (methods not claimed) as described herein, includes methods (methods not claimed) for effectively introducing larger diameter catheter systems into smaller arteries at the artery access stage of endovascular procedures including neuro-, cardio-, body-, and/or peripheral-intervention procedures.

### Scope of Language

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items.

Spatially relative terms, such as "distal", "proximal", "forward", "rearward", "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a feature in the figures is inverted, elements described as "under" or "beneath" other elements or features would then be oriented "over" the other elements or features. Thus, the exemplary term "under" can encompass both an orientation of over and under. A feature may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

It will be understood that when an element is referred to as being "on", "attached" to, "connected" to, "coupled" with, "contacting", etc., another element, it can be directly on, attached to, connected to, coupled with or contacting the other element or intervening elements may also be present. In contrast, when an element is referred to as being, for example, "directly on", "directly attached" to, "directly connected" to, "directly coupled" with or "directly contacting" another element, there are no intervening elements present.

It will be understood that, although the terms "first", "second", etc. may be used herein to describe various elements, components, etc., these elements, components, etc. should not be limited by these terms. These terms are only used to distinguish one element, component, etc. from another element, component. Thus, a "first" element, or component discussed herein could also be termed a "second" element or component without departing from the teachings. In addition, the sequence of operations (or steps) is not limited to the order presented in the claims or figures unless specifically indicated otherwise.

Other than described herein, or unless otherwise expressly specified, all of the numerical ranges, amounts, values and percentages, such as those for amounts of materials, elemental contents, times and temperatures, ratios of amounts, and others, in the following portion of the specification and attached claims may be read as if prefaced by the word "about" even though the term "about" may not expressly appear with the value, amount, or range. Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques.

Unless otherwise defined, all technical and scientific terms used herein have the same meaning as commonly understood by one of ordinary skill in the art.

Various aspects of the systems and methods (methods not claimed) will now be described with reference to the figures. For the purposes of illustration, components depicted in the figures are not necessarily drawn to scale. In particular, directions of width and length may be distorted with respect to one another in that widths generally reflect the internal diameters of arteries (typically mm scale) whereas lengths reflect the lengths of arteries (typically a cm+ scale). Thus, for clarity, the "length" scale is generally (but not necessarily) compressed with respect to the width scale and/or shows breaks in the length of components. As such, emphasis is placed on highlighting the various contributions of the components to the functionality of various aspects of the systems and methods (methods not claimed). A number of possible alternative features are introduced during the course of this description. It is to be understood that, according to the knowledge and judgment of persons skilled in the art, such alternative features may be substituted in various combinations to arrive at different embodiments.

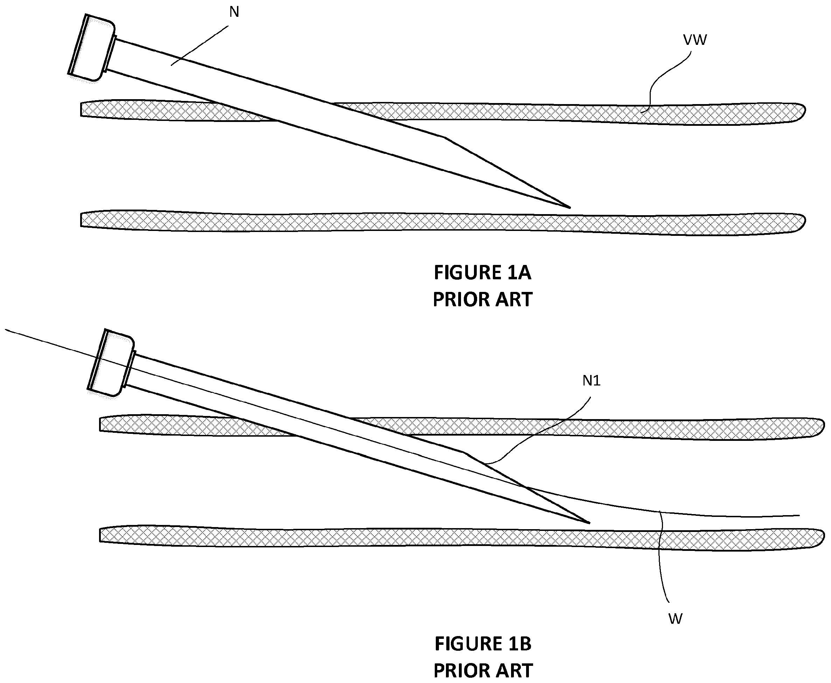

With reference to the figures, systems and methods (methods not claimed) for introducing catheters into arteries are described. Figures 1A-1G illustrate known steps of gaining access to an artery and are provided for background. While the description generally focusses on radial artery access, it is understood that the apparatus and methods (methods not claimed) described herein can apply to other access arteries including the femoral artery and carotid artery.

In a first step, as shown in Figure 1A a hollow bore needle N is used to puncture an artery. The needle has a typical outer diameter of about 1mm and internal diameter of about 0.5 mm.

As shown in Figure 1B, a wire W is introduced into the needle such that it feeds through the needle and out of the distal tip N1 of the needle. The wire will have a diameter that allows its passage through the needle.

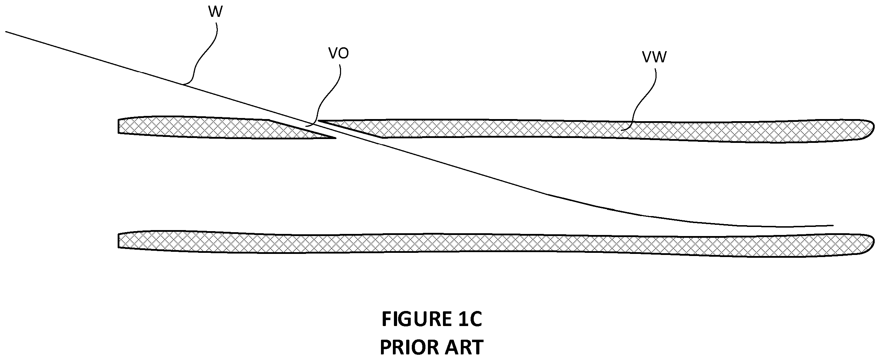

As shown in Figure 1C, the needle is then withdrawn over the wire leaving the wire in place in the vessel and protruding through the vessel opening VO.

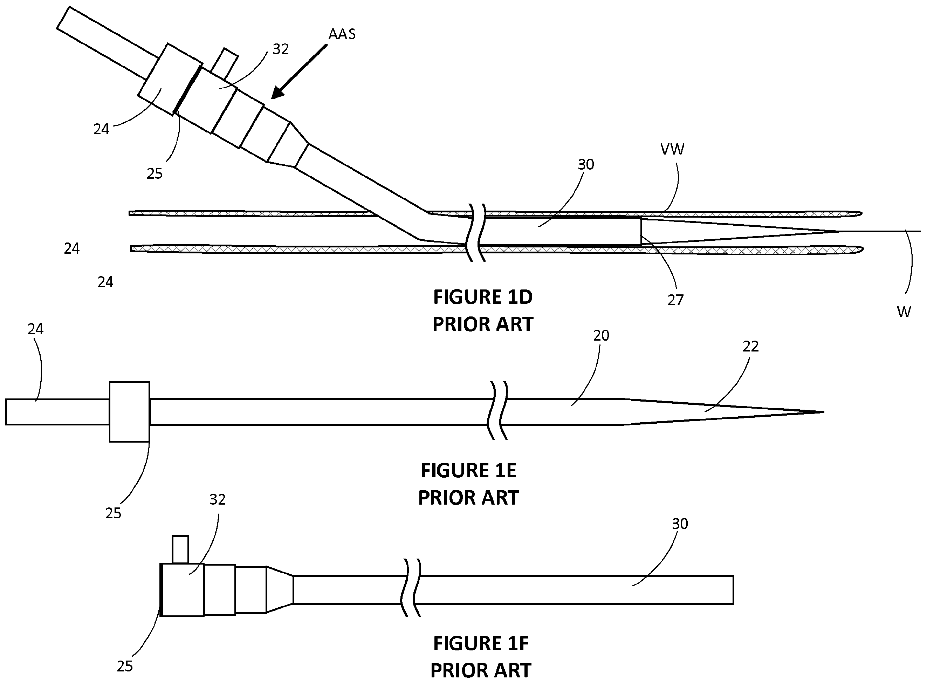

An arterial access system (AAS) as shown in Figures 1D, 1E and 1F, is guided over the wire and into the vessel through the VO. In accordance with the prior art, the AAS typically includes an internal guide/introducer 20 having a pointed distal tip 22 and an external sheath 30. Both the internal guide and the external sheath have known extracorporeal connectors 24, 32 that during insertion into the BV are connected at junction 25 allowing the assembled AAS to be inserted together. The overall length of an internal guide 20 is typically in the range of 15cm and the external sheath 30 typically has an overall length of about 12 cm.

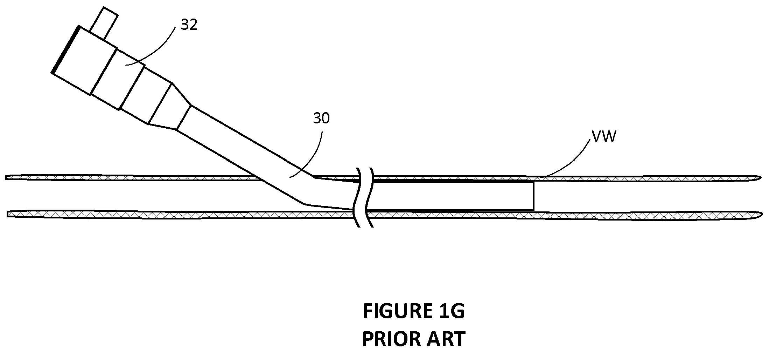

After the AAS has been introduced as shown in Figure 1D, the internal guide 20 and wire W are removed (Figure 1G) thus providing a conduit into the vessel through the external sheath 30 that allows fluids and/or instruments to be introduced into the vessel through the AAS.

The inventors recognized that the introduction of a larger bore catheter such as an aspiration catheter (AC) suitable for aspirating a clot from the cerebral arteries would be too large to be introduced into smaller vessels (such as the radial or brachial arteries) within an external sheath 30. That is, the external sheath of a radial artery AAS has a practical maximum internal diameter of about 6F (OD of about 7.2F) to allow the passage of a 6F AC through the external sheath whereas it is desirable to introduce ACs into the cerebral vessels that have outside diameters greater than 6F (eg. about 6-8F).

Hence, there has been need for arterial access systems (AASs) that enable the introduction of larger catheters such as an aspiration catheter (AC) into the radial arteries. AASs are described herein with reference to ACs and cerebral access procedures, although it is understood that different types of catheters for different procedures are contemplated.

As described in co-pending applications, US provisional application 62/878,652 filed July 25, 2019, US provisional application 63/029,401 filed May 23, 2020 and International patent application PCT/CA2020/051026 filed July 24, 2020, the design of an aspiration catheter that can be maneuvered from the arterial access point (e.g. groin or radial artery) is characterized by a soft distal tip section that is both sufficiently soft to be atraumatic as it navigates through the cerebral arteries, flexible enough to pass through tight curves and also allow effective suction to be applied to a blood clot. Also, the tip is radio-opaque to allow visualization during navigation through neck and intracranial vessels.

Importantly, in order to ensure that the AC is suitable for aspiration, the AC tip generally cannot have a taper at its distal tip (i.e. a narrower wall thickness at the distal tip tapering to a wider wall thickness in the proximal direction) as an AC requires a certain radial stiffness to prevent the distal tip from collapsing when suction is being applied to a clot.

It is also important that when an AAS is being deployed through a VO that the risk of damaging the vessel wall is minimized. Thus, while equipment being pushed into a vessel can tightly engage the vessel, AASs have generally been designed such that the external sheath has an internal taper that enables a smooth transition between the internal guide and external sheath without a significant edge at point 27 (see Figure 1D). The absence of a significant edge at the boundary between the internal guide and external sheath allows the external sheath to be pushed through the VO while engaged with the vessel wall without damaging the vessel wall.

The inventors also realized it would be desirable to be able to introduce an AC into a radial artery with the AC being supported internally by an internal guide 20 with the tapered tip 22 of the internal guide protruding from the end of the AC. However, the inventors recognized that the transition between the internal guide 20 and AC is problematic due to the oblique shape of the distal tip of an AC, the softness/flexibility of the distal tip of the AC, the presence of a radio-opaque marker and the lack of distal taper. In other words, without a substantially equivalent stiffness as a comparable external sheath (as shown in Figure 1F), the distal tip of an AC supported by an internal guide alone has a tendency to crumple and/or buckle with respect to the internal guide when the two are combined as an internal guide and AC. In particular, this problem occurs when such an assembly is being pushed through the vessel opening (VO) and for a number of centimeters upstream of the VO.

The inventors recognized that in order for the soft distal tip of an AC to pass through and past the VO, a portion of the distal tip of the AC must be protected for its passage through the first 0-15+ cm (approximately) of the vessel as the surgeon is pushing the AC upstream, for example, towards the aortic arch. Generally, internal support in the form of an introducer is not needed after the initial approximate 15 cm as the arteries become sufficiently large that the AC tip will not be tightly engaged against the VW and will be able to be pushed forward without internal support and without crumpling.

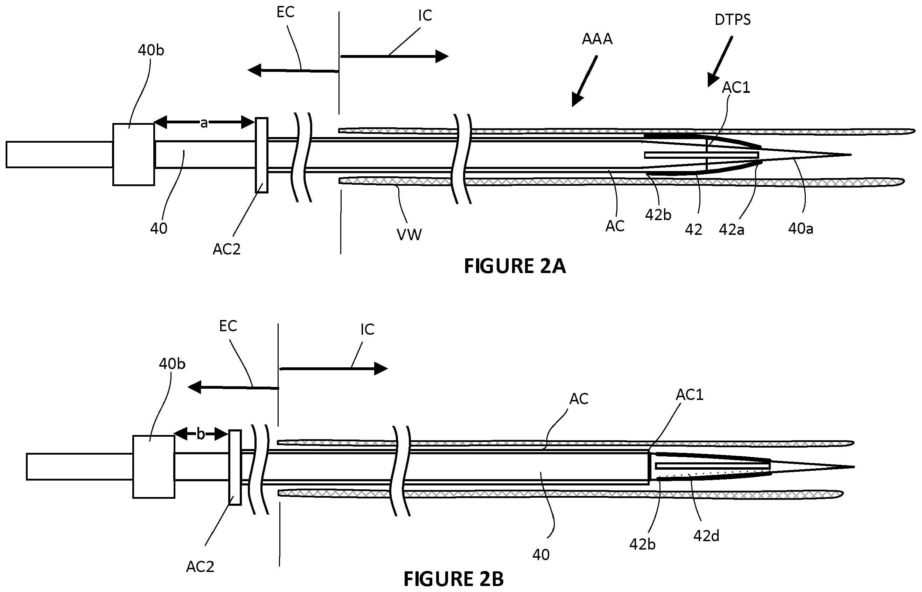

Accordingly, in a first embodiment as shown in Figures 2A-2C, a system (referred herein as an arterial access system (AAS)) for advancing an AC through a VO and into such smaller arteries is described.

In Figure 2A, an assembled AC and internal guide 40 having an AC distal tip protection system (DTPS) is shown (i.e. an AAS). The internal guide 40 is similar in design to prior art internal guides 20 insomuch as it has a tapered distal tip 40a and an internal bore allowing a wire to pass through its center. In contrast to past internal guides, the length is sufficient to extend the full length of an AC (or equivalent) and protrude beyond the proximal end of the AC. Figures 2A-2C show an assembled AC/internal guide assembly (AAS) pushed through a VO and a short distance into an intracorporeal zone (IC) of a vessel as well as extracorporeal zone (EC) where the AAS can be manipulated.

In greater detail, the internal guide 40 is sufficiently long to extend beyond the proximal end of the AC and be capable of manipulation from the proximal end of the AC. In addition, the internal guide 40 includes the DTPS that is formed as part of the internal guide to protect and prevent buckling of the AC tip as it is being inserted through the VO and into the artery.

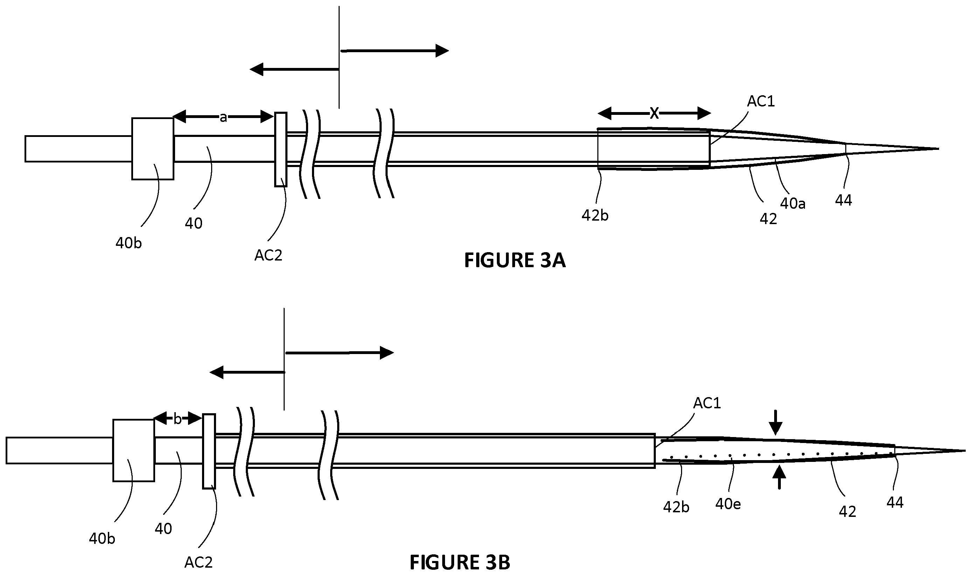

As shown in Figures 2A and 3A, in various embodiments, the DTPS includes a plurality of biased or elastic arms (Figure 2A) or sheath (Figure 3A) 42 configured to the internal guide 40 adjacent and/or forming part of its tapered surface 40a that function as a protective sheath for the distal tip AC1 of the AC during the critical steps of passing the distal tip AC1 of the AC through the skin (i.e. VO) and pushing it forward through the narrowest portions of a vessel. The elastic arms/sheath 42 provide partial or full circumferential cover to the distal tip AC1 of the AC whether a single body or multiple arms.

The DTPS has a distal end 42a secured to the internal guide 40 and a proximal end 42b that covers the distal tip AC1 of the AC. The DTPS extends proximally a sufficient distance to frictionally engage over a sufficient length of the AC so as to prevent separation/buckling of the AC1 with respect to the internal guide 40.

As shown schematically, the proximal end of the AC includes a stop AC2 that defines a proximal end of the AC. Similarly, the internal guide 40 includes a proximal end stop 40b. The end stop 40b and AC2 are separated a short distance shown as "a" in Figures 2A and 3A. As can be understood, by manipulating both the end stop 40b and AC2 with respect to one another, the AC and internal guide 40 can telescopically move with respect to one another.

As shown in Figure 2B, the end stop 40b and AC2 have been moved towards one another to a distance "b". As can be seen, this has moved the internal guide 40 distally with respect to the AC such that the proximal edges 42b of the elastic arms 42 are distal to AC1. Distance "b" can be zero where the end stop 40b and AC2 abut one another.

The internal guide 40 can be provided with one or more recesses 42d within the internal guide 40 such that as the elastic arm ends 42b of DTPS move past AC1, they will be drawn into the recess(es) 42d (dotted line) and thus become flush (or recessed) with respect to the external surface of the internal guide 40.

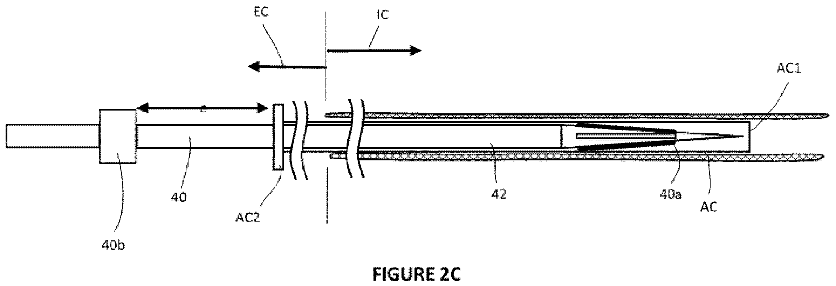

Thereafter, the end stop 40b can be moved proximally to a length greater than "a" (shown as "c") and relative to AC2 such that the internal guide 40 can be withdrawn from the AC with the DTPS being able to pass into AC through AC1 (Figure 2C).

The internal guide 40 can then be fully removed from the AC thus having introduced the AC into the vessel and allowing further steps of the procedure to be completed.

Typically, the AC/internal guide system (AAS) would be pushed forward a distance up to about 15cm from the VO before conducting the steps as described above.

Figures 3A-3D show a different embodiment of the DTPS. In this embodiment, the DTPS is a resiliently flexible sheath or "umbrella" 42 that is attached to the tapered surface 40a of the internal guide 40 at point 44. Initially, at the start of the procedures, umbrella 42 extends over AC1 and a distance X sufficient to frictionally retain the umbrella 42 over AC1. The umbrella may be an elastic material.

In this embodiment, after the AC/internal guide assembly has been inserted into the vessel, the internal guide 40 is removed following similar steps to those described above. That is, the internal guide is initially pushed distally to push the umbrella past AC1 (Figure 3B) such that AC1 is uncovered. Depending on the design of the umbrella, the umbrella may elastically contract (as shown by opposing arrows in Figure 3B) over the internal guide and within an appropriate recess 40e on the internal guide 40 to become flush with the internal guide 40 as shown in Figure 3B.

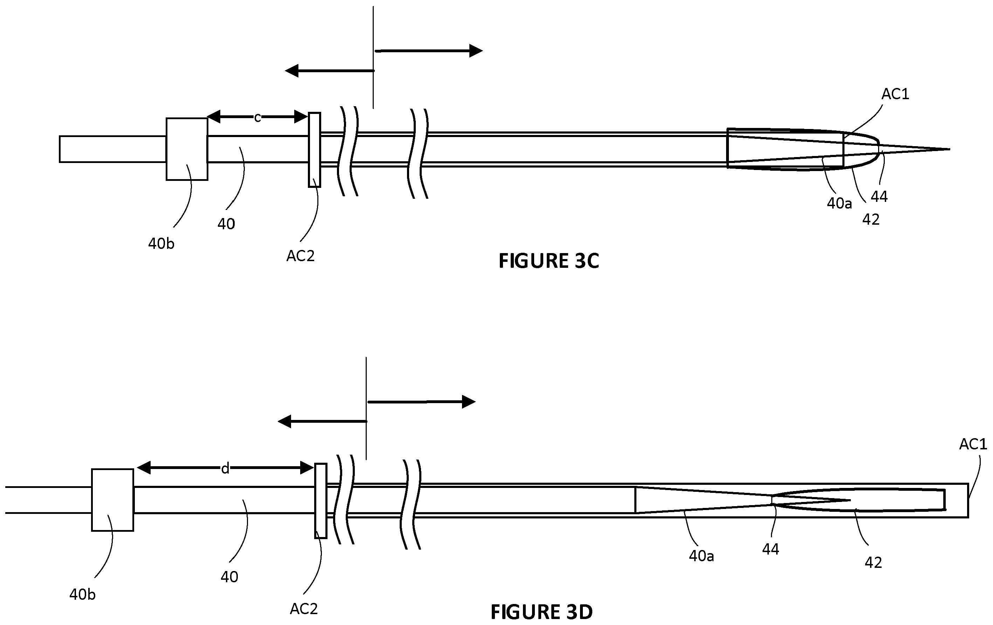

In another embodiment, the umbrella 42 may "invert" with respect the internal guide 40 when subsequently withdrawn as shown in Figures 3C and 3D. Figure 3C shows the umbrella beginning to invert and Figure 3D shows the umbrella inverted and being withdrawn into the AC. Inverting the umbrella may occur by pushing the internal support forward to disengage the umbrella from the AC or by simply pulling back on the AC.

Accordingly, as above, the internal guide can then be fully withdrawn.

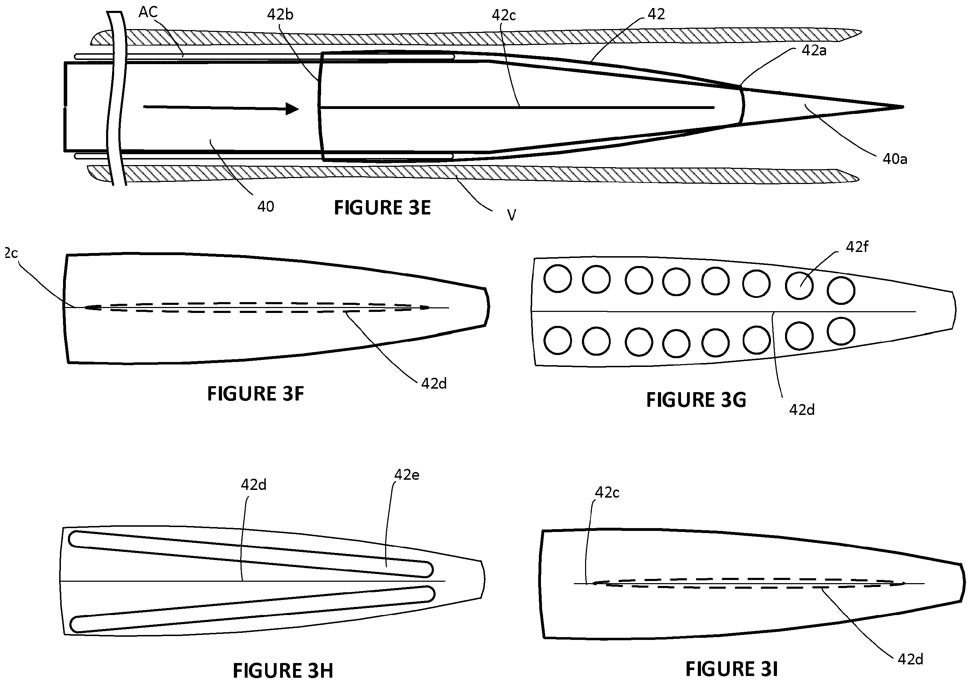

The umbrella 42 can be configured to the internal guide with a number of designs that enable effective inversion of the umbrella. Importantly, the umbrella requires properties that enable it a) to invert without getting stuck as the umbrella turns inside out and b) in a manner that doesn't damage the AC soft tip or vessel intima during this process. Specifically, as the body of the umbrella is inverting over and around the distal tip of the AC, it must be able to bend through 180 degrees while sliding over and into the distal tip of the AC. In addition, the portion of the umbrella overlapping the outer distal tip of the AC must be able to slide distally over the AC (and against the vessel wall) without undue friction until the point that the umbrella is fully inverted and within the AC. These functions can be achieved with various designs as described with reference to Figures 3E to 3I.

Figure 3E shows an internal guide, AC and an umbrella engaged over the AC and Figures 3F-3I show various embodiments of the umbrella 42. As shown in Figure 3E, the umbrella 42 is engaged over the distal tip of the AC to allow distal movement of the assembly through a vessel V. The umbrella includes at least one longitudinal slit 42c extending from the proximal edge 42b towards the distal edge 42a which allows the umbrella to open (i.e. spread apart). For example, the umbrella may be provided with a single slit (i.e. the umbrella is a "cape") or two slits (i.e. the umbrella has "leaflets"). In the one-slit or two-slit embodiment, the slit(s) allows the proximal edge of the umbrella to open as the internal guide is being withdrawn thus relieving friction around the distal tip of the AC during the inversion.

Tight circumferential engagement of the proximal edge of the umbrella against the AC is not necessarily required on its own as the surgeon can ensure that the umbrella remains engaged over the AC during the steps of inserting the internal guide, AC and umbrella into the VO such that as the tip of AC passes through the VO the umbrella is engaged over the distal tip of the AC.

Preferably, when assembled, the umbrella will overlap with the AC over a distance of 1-5 cm and preferably about 2-4 cm. Generally, as explained below, when there is about 1-5cm of overlap, the surgeon can ensure that the umbrella remains properly engaged with the AC as the assembly is inserted.

More specifically, after vessel puncture, the surgeon will insert the assembled internal guide, AC and umbrella into the VO. As the tip of the internal guide is inserted, the surgeon can squeeze/hold the proximal end of the umbrella over/on the AC as the assembly is being inserted. After the internal guide tip 40a has been inserted, the non-tapered portion of the assembly will quickly engage with the vessel wall thus keeping the umbrella tightly engaged over the AC. By applying a gentle squeezing pressure over the assembly, the surgeon can ensure that both the internal guide 40 and AC are not slipping with respect to one another as the assembly is advanced. As described above, the assembly is advanced up to about 15cm (6 inches) into a vessel that is tightly engaged with the assembly; hence, the umbrella will not separate from distal tip of the AC and protect the tip of the AC.

When the assembly is in its distal position, as described above, the surgeon can hold the AC at that position and then pull back on the internal guide to invert the umbrella and remove the internal guide. As the internal guide is being pulled back, the proximal edge of the umbrella is not tightly engaged (i.e. circumferentially) with the AC and can thus spread apart and slide distally over the AC.

As a key objective is to provide a structure that allows the umbrella to be inverted, other embodiments that reduce the total volume of umbrella material are contemplated that can assist in enabling the umbrella to make a 180 degree turn over the distal tip of the AC and otherwise reduce friction during this process.

Figures 3F - 3I show different embodiments of umbrellas that may be attached to the distal tip of an internal guide and where the volume of umbrella material has been reduced through the incorporation of various voids such as holes (Figure 3G), and/or longitudinal slots (Figure 3H) in the body of the umbrella.

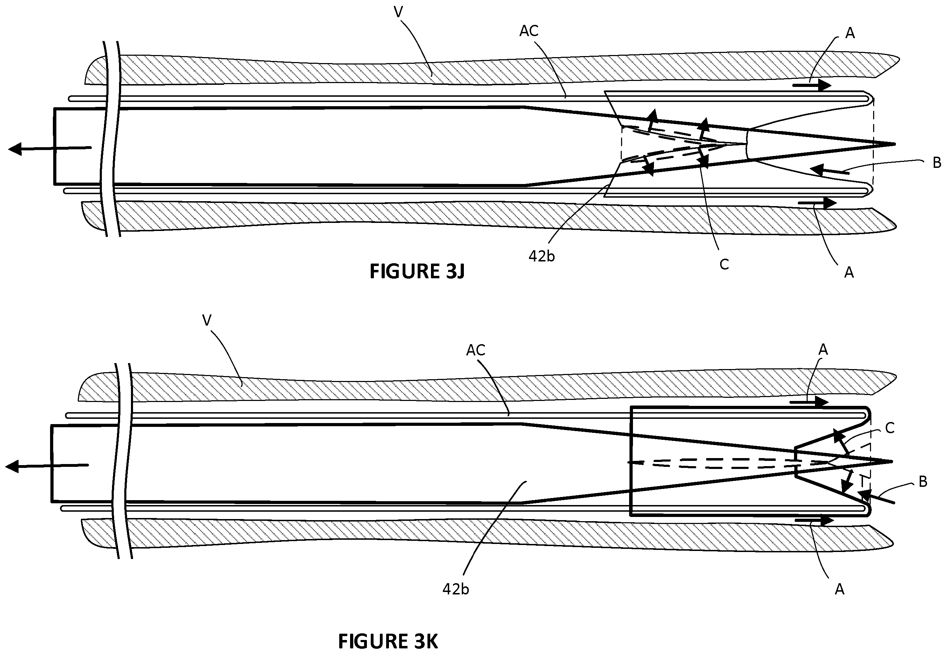

In one embodiment as shown in Figure 3F, the umbrella slits may be joined but include one or more zones of weakness 42d that will fail during the inversion procedure allowing the slits to open as necessary. Such zones of weakness may be narrow regions of material that will tear as the inversion process is initiated or other systems such as water-soluble glues or polymers. Depending on the particulars of a design and as shown in Figures 3J and 3K, umbrella sections may be designed to open from the proximal end as shown in Figure 3J or the distal end as shown in Figure 3K or may inherently open from one side or the other.

Figure 3J shows an umbrella partially through the inversion process where the proximal portion of the umbrella is sliding forward (Arrows A) over the AC and distal portion of the umbrella has already inverted and has been drawn into the AC (Arrow B). Arrows C show the general direction that the umbrella may open from the proximal end 42b of the umbrella.

Figure 3K also shows an umbrella partially through the inversion process where the umbrella is opening from the distal portion. As above, the proximal portion of the umbrella is sliding forward (Arrows A) over the AC and distal portion of the umbrella has already inverted and has been drawn into the AC (Arrow B). Arrows C show the general direction that the umbrella may open from the proximal end 42b of the umbrella.

In further embodiments, the slits may not extend fully to the proximal edge of the umbrella as shown in Figure 3I. In these embodiments, sufficient circumferential friction along the length of the umbrella may be reduced by such slits to enable umbrella inversion whilst allowing pre-assembly of a catheter and umbrella on an internal support.

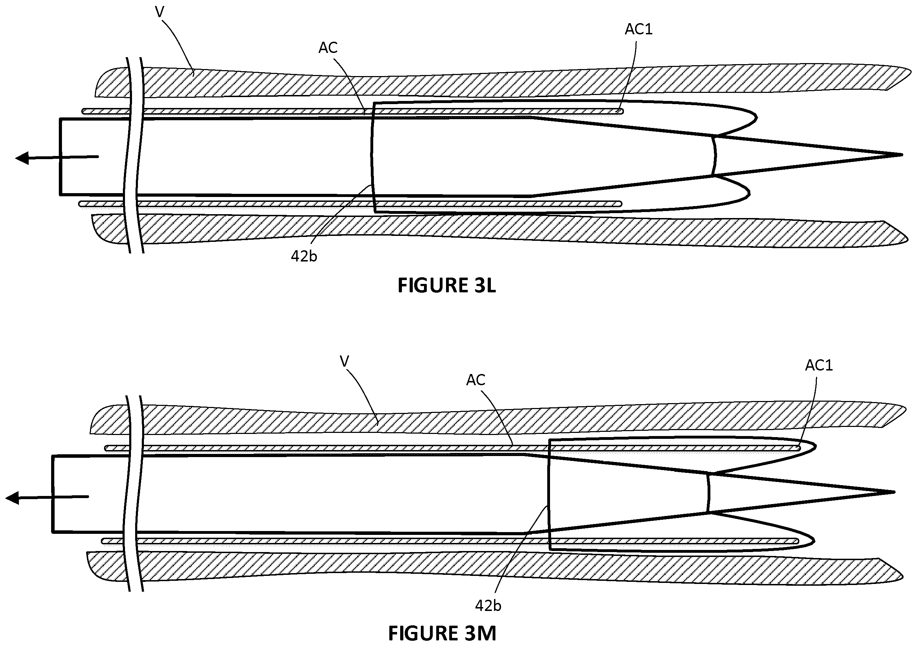

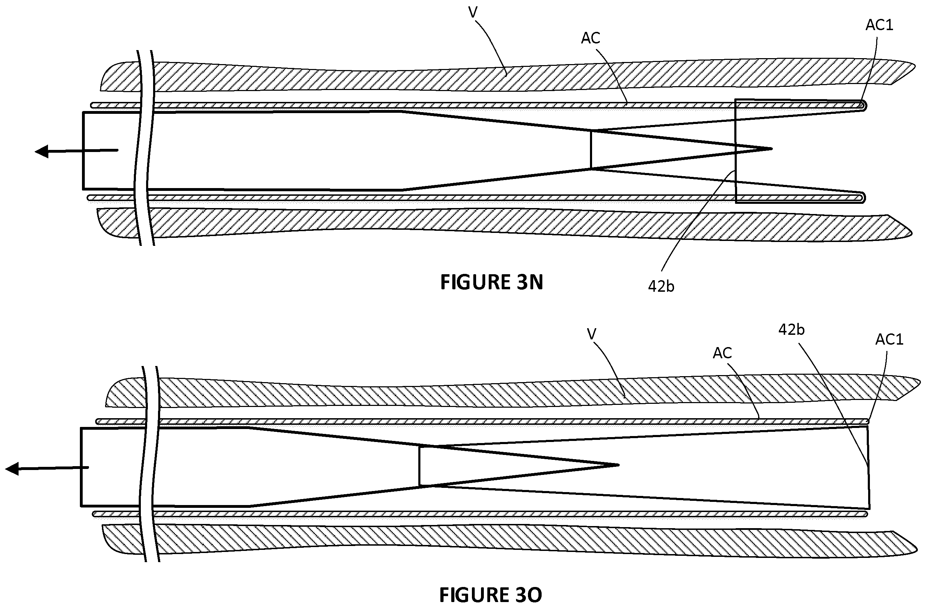

Figures 3L to 3O show additional sketches showing of the steps of inverting the umbrella. As shown, the distal edge of the umbrella is preferably located on the distal tip of the internal guide where its diameter is approximately 50% of its widest diameter in order to provide some space for the umbrella as the inversion is initiated. Figure 3L shows a first step of inversion where the umbrella has started to invert but has not yet wrapped over the distal tip AC1. Figure 3M shows the umbrella beginning to be engaged with AC1, Figure 3N shows the umbrella inverting over AC1 with the proximal edge 42b sliding forward and Figure 3O shows the umbrella fully inverted.

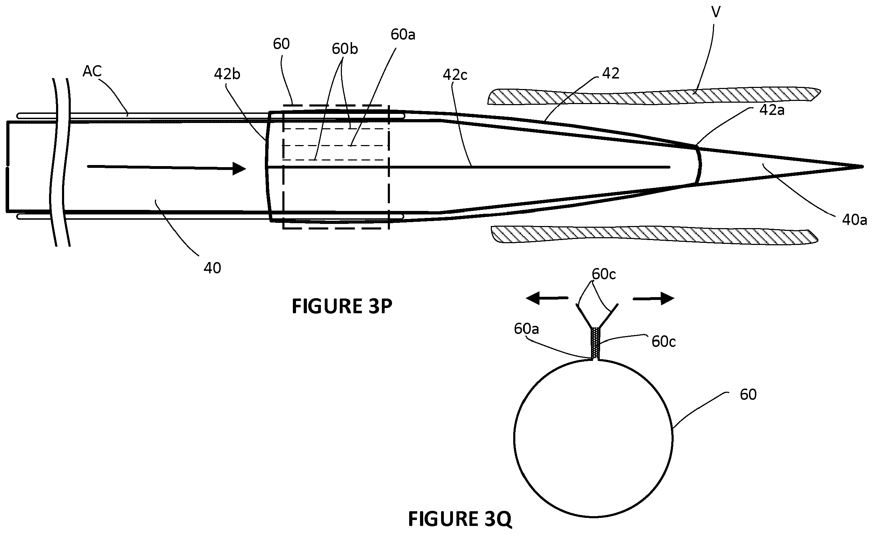

In another embodiment as shown in Figures 3P and 3Q, the proximal end of the umbrella is held closed by a small removable band 60 (shown in dotted lines in Figure 3P) that can be removed shortly before the proximal end of the umbrella enters the vessel. As shown, the band 60 may have an edge/seam 60a together with one or more pull tabs 60b that allows band to be removed. As shown in Figure 3P, two sides of the band are held together by a small adhesive band 60c that can be separated by pulling the tabs 60b in opposite directions just as the umbrella is about to be inserted into the vessel. This design can enable pre-assembly of the catheter and internal support at the factory.

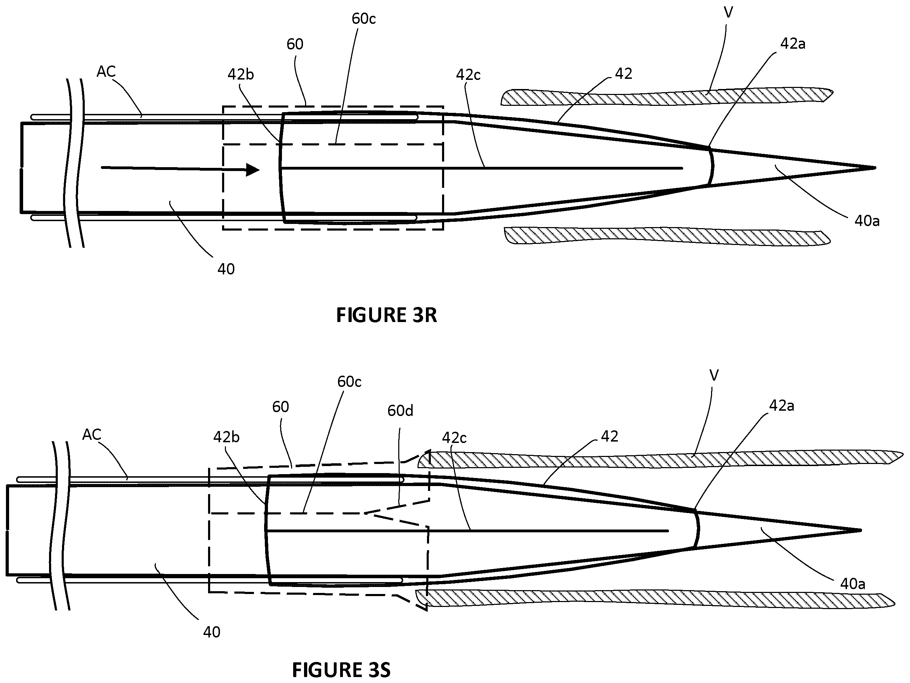

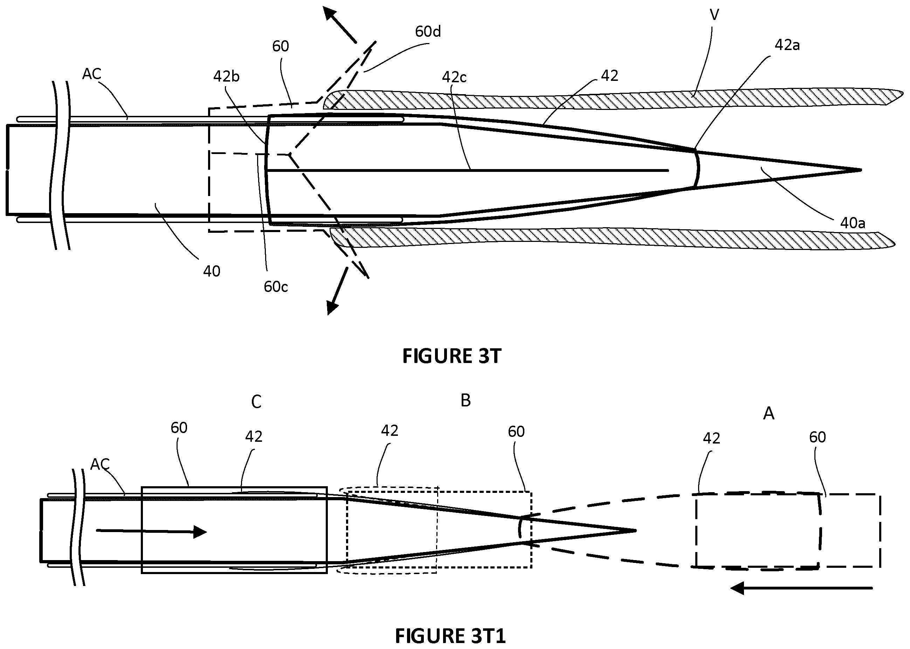

Figures 3R-3T show another embodiment where the removable band 60 secures the umbrella over the AC and is torn away along a seam 60c as the AAS is entering the vessel. Figure 3R shows the band fully engaged over the umbrella 42 and AC with the distal tip of the AAS having entered a vessel. As the distal edge of the band 60 engages with the vessel, the band 60 does not enter the vessel and is torn away 60d along seam 60c as shown in Figures 3S and 3T.

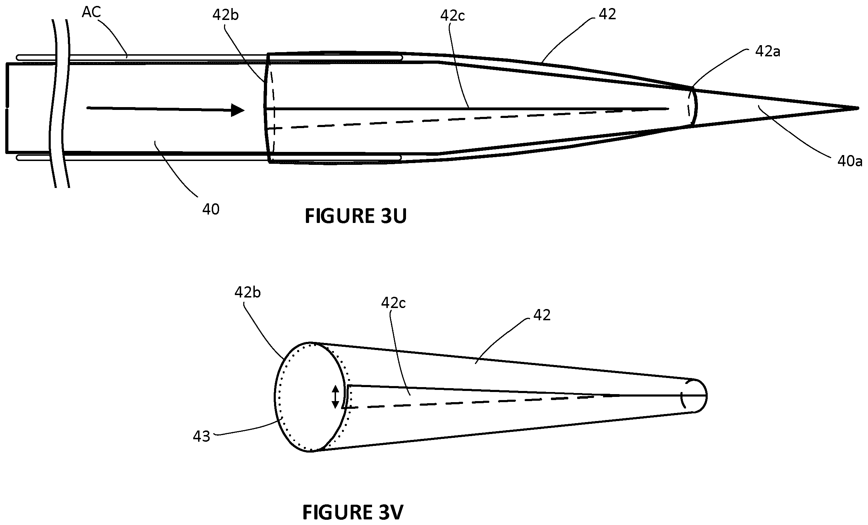

Figure 3U shows another embodiment where the umbrella 42 has a shaped band 43 configured to the proximal edge 42b. In this embodiment, the shaped band has a preformed shaped configured for engaging with the AC to hold the umbrella in its closed position. The shaped band may be a memory metal (such as nitinol) or a polymeric band having thermo properties that will relax spring pressure upon exposure to body temperatures thus allowing the shaped band to relax and allow the umbrella to slip over the AC. As shown in Figure 3V, the umbrella may be provided with a small amount of overlap. The shaped band must be sufficiently pliant to enable its passage through the AC once the umbrella has inverted.

The umbrella, regardless of the embodiment, is preferably manufactured from materials such as expanded polyflurotetraethylene (PTFE) which provides sufficient lubricity and durability during a procedure to effectively enable the AC to be advanced but is also permeable to gas to enable sterilization during manufacture.

### Assembly

The arterial access assemblies described above may be assembled at a factory or at a treatment center immediately prior to use.

In the case of factory assembly, kits including various catheters and internal guides may be packaged together. Typically, after manufacture of the internal guide with a DTPS and the catheter, both having appropriate internal and external diameters for engagement with one another, the two components would be assembled such that the DTPS is properly engaged with the distal end of the catheter. After assembly and sterilization, the AAS would be packaged in a single package for delivery and subsequent use at a treatment facility.

As understood by those skilled in the art, various combinations of catheters and internal guides may be assembled based on the properties of specific catheters and their diameters.

For example, a factory assembled kit may include any one of a 6-8F AC having particular functional properties for cerebral endovascular procedures configured to an appropriately sized internal guide.

Practically, as physicians may desire to use particularly brands of catheters, it may not be commercially feasible for the manufacturer of internal guides to assemble internal guides for a wider range of catheters. As a result, assembly of internal guides with physician selected catheters at a treatment center is desirable.

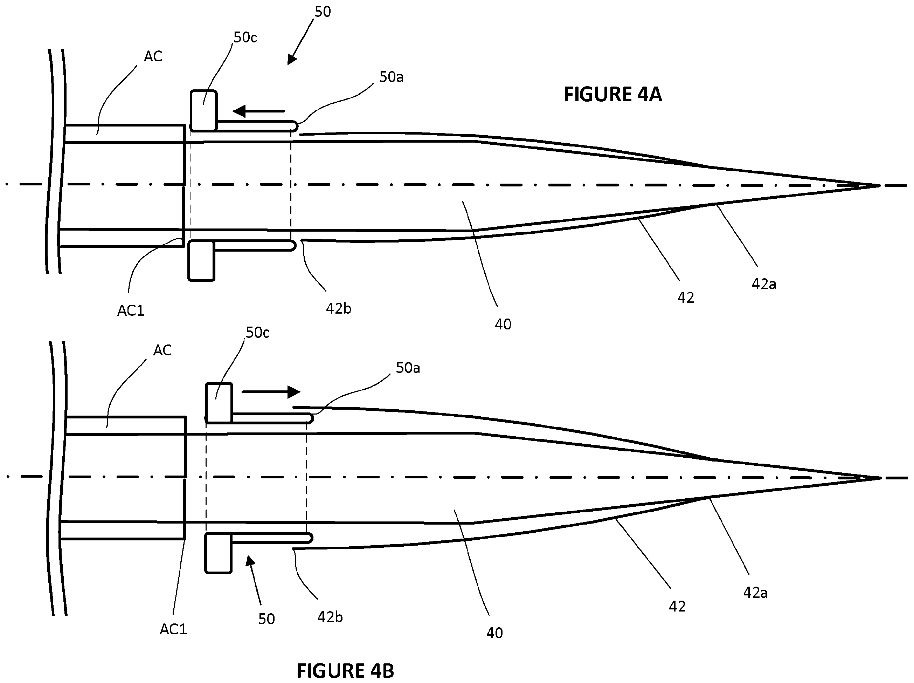

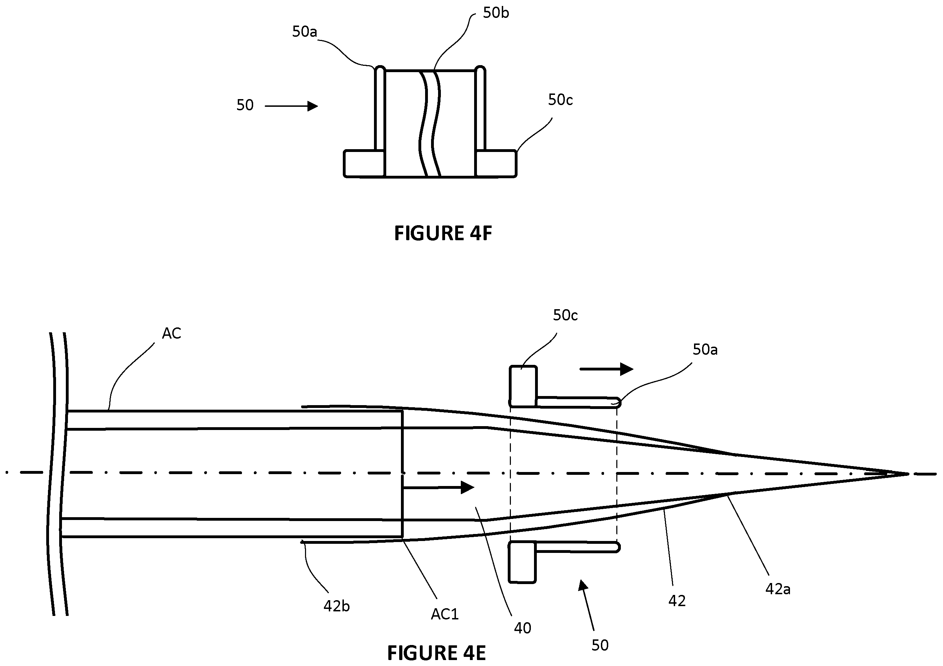

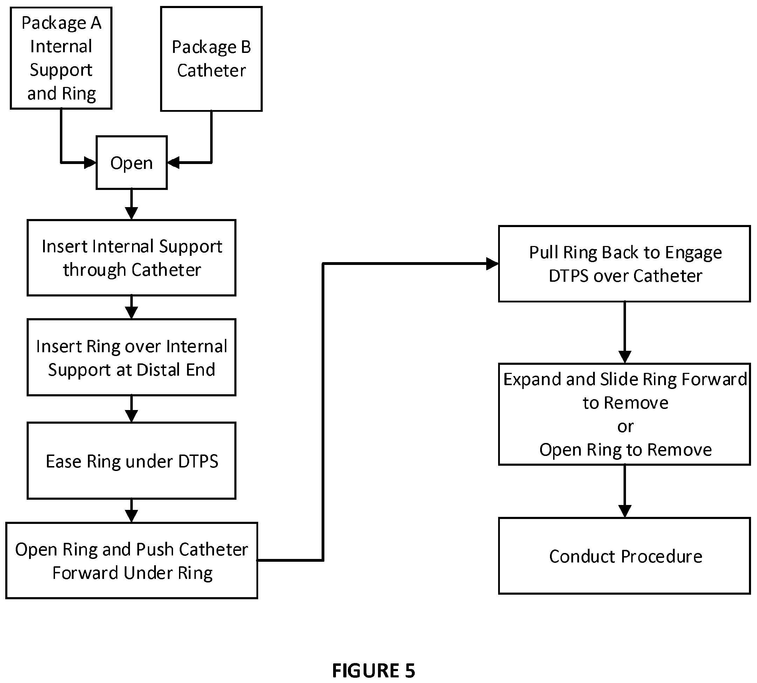

As shown in Figures 4A-4F and Figure 5, systems and methods (methods not claimed) for assembly of an internal guide 40 with an AC are described.

In accordance with one embodiment, the following steps may be followed to assemble an internal support 40 manufactured and packaged (referred to as Package A) within sterilized packaging with a catheter (AC) from another manufacturer, packaged and sterilized within separate packaging (referred to as Package B).

Package A containing an internal support 40 and a ring 50 from one supplier/manufacturer is selected. The internal support 40 has a displayed outside diameter (OD) and length. The ring 50 has an inside diameter (ID) substantially corresponding to the internal support OD and able to slide over the internal support.

Package B containing a catheter having a known OD, ID and length is selected. The catheter may be from a different supplier/manufacturer.

The internal support 40 of package A has a length longer than the length of the catheter in package B. Package A may also include a wire.

Both packages are opened and the distal end of the internal support 40 is inserted into the proximal end of the catheter through the proximal end of the catheter until it extends from the distal end of the catheter. The DTPS 42 of the internal support 40 is pushed past the distal end of the catheter AC1, a distance sufficient to allow the ring to be placed over and proximal to the DTPS.

The ring 50 is slid over the distal tip of the internal support 40 and placed proximal to the DTPS as shown in Figure 4A and then moved distally such that the distal edge 50a of the ring is worked under the proximal edge 42b of the DTPS. A separate edge lifting device (e.g. a non-traumatic spatula; not shown) may be utilized to assist in lifting the DTPS away from the internal support 40 such that the proximal edge 42b fully surrounds the distal edge 50a of the ring as shown in Figure 4B.

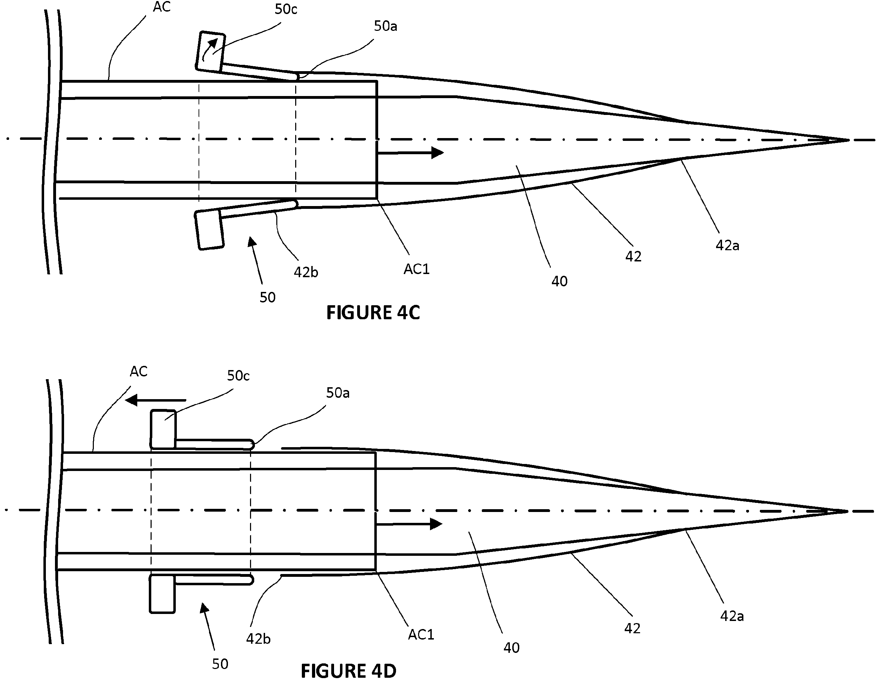

As shown in Figures 4C, 4D and 4F, ring 50 is elastically expandable or openable having at least a portion of the ring with a flexible portion or openable junction 50b enabling expansion or opening of the OD of the ring. Ring 50 is preferably provided with flange 50c enabling a user to hold and/or apply pressure to the ring.

As shown in Figure 4C, catheter AC is pushed forward and flange 50c manipulated to open the ring 50 to enable the AC to insert within the ring 50 thus causing the DTPS to expand over the AC. The AC is pushed forward a sufficient distance for the DTPS to overlap the AC and frictionally engage with the distal tip region of the DTPS. As shown in Figure 4D, the ring is then pulled back from the DTPS such that the DTPS engages over the AC and disengages with the DTPS.

As shown in Figure 4F, the ring is then opened and pushed forward over the AC and DTPS to remove the ring from the assembly. Alternatively, the ring may be opened at one or more junctions 50b to allow the ring to be removed.

The combined AC and internal support can then be introduced into an artery via the procedures described above.

Sterilization of the internal support and DTPS is an important consideration. Hence, it is desirable that the DTPS is manufactured from materials allowing appropriate sterilization to be conducted prior to packaging. Expanded poly tetrafluoroethylene (EPTFE) can have sufficient porosity to enable sterilizing gases such as ethylene oxide to fully and properly penetrate the structures for sterilization.

In those embodiments where the sheath/umbrella includes one or more slits that extend across a proximal edge of the umbrella, assembly may be conducted within the operating room by the physician and/or their assistants. Such assembly may be facilitated by the umbrella DTPS delivered in the inverted state and covered with a sleeve. The umbrella sheath and sleeve would then be proximally introduced into the AC, the sleeve would then be translated proximally, facilitating the positioning of the umbrella DTPS over the AC tip, and finally the sleeve would be peeled away and discarded as shown in Figure 3T1 in steps A, B and C.

Although the technology been described and illustrated with respect to various embodiments and preferred uses thereof, it is not to be so limited since modifications and changes can be made therein which are within the full, intended scope of protection as defined in the appended claims.

## Claims

1. A system for introducing a catheter into a vasculature through a vessel opening (VO) comprising:
a catheter having an internal diameter and external diameter;
an internal support (40) sized for telescopic movement within the catheter, the internal support (40) for supporting the catheter and protecting a distal tip of the catheter as the catheter is introduced through a VO, the internal support (40) having:
a tapered distal tip (40a) for introducing the system through the VO; and,
a protective cover (42) connected to the tapered distal tip (40a) extending proximally for engagement over the distal tip of the catheter, the protective cover (42) moveable between an engaged position over the catheter and a disengaged position;
wherein selective movement of the internal support (40) relative to the catheter causes the protective cover (42) to move from the engaged position to the disengaged position and when in the disengaged position allows the internal support (40) and protective cover (42) to be proximally withdrawn through the catheter; and,
wherein the protective cover (42) is a sheath and the sheath inverts to the disengaged position during withdrawal of the internal support (40) and where the sheath has at least one slit (42c) extending along a longitudinal length of the sheath, the slit (42c) for relieving friction between the catheter and the internal support (40).

2. The system as in claim 1 wherein the catheter has a catheter end stop and the internal support (40) has an internal support end stop (40b) and wherein when the protective cover (40) is in the engaged position, movement of the internal support end stop (40b) towards the catheter end stop causes the protective cover (42) to move to the disengaged position.

3. The system as in any one of claims 1- 2 where the catheter is an aspiration catheter having a soft distal tip region and a length sufficient to extend from a radial artery VO to cerebral vessels for a treatment of ischemic stroke.

4. The system as in claim 3 where the aspiration catheter is 5-8F or 6-8F.

5. The system as in any one of claims 1-4 where the internal support (40) is hollow, and the tapered distal tip (40a) includes a through bore allowing the internal support (40) to ride over a wire.

6. The system as in claim 1 where the at least one slit (42c) extends to a proximal edge of the sheath.

7. The system as in claim 1 where the at least one slit (42c) includes a zone of weakness capable of opening during inversion.

8. The system as in claim 1 where the at least one slit (42c) does not extend across a proximal edge of the sheath.

9. The system as in claim 1 where the at least one slit (42c) is a longitudinal slot.

10. The system as in claim 1 where the sheath includes one or more voids to reduce a total volume of the sheath and facilitate sheath inversion during withdrawal of the internal support.

11. The system as in any one of claims 1-10 further comprising a band (60) surrounding a proximal end of the sheath for holding the sheath on the catheter prior to use.

12. The system as in claim 1 wherein a proximal edge of the protective cover (42) includes a shaped band (43) for applying a biasing force over the catheter to hold the protective cover (42) in the engaged position on the catheter.

13. The system as in claim 12 wherein the shaped band (43) is a thermo material and wherein exposure to a body temperature relaxes the biasing force.

14. A kit comprising:
an internal support (40) within sterilized packaging, the internal support (40) sized for telescopic movement within a catheter, the internal support (40) for supporting the catheter and protecting a distal tip of the catheter as the catheter is introduced through a VO, the internal support (40) having:
a tapered distal tip (40a) for introducing the kit through the VO; and,
a protective cover (42) connected to the tapered distal tip (40a) extending proximally for engagement over the distal tip of the catheter, the protective cover (42) moveable between an engaged position over the catheter and a disengaged position;
wherein selective movement of the internal support (40) relative to the catheter causes the protective cover (42) to move from the engaged position to the disengaged position and when in the disengaged position allows the internal support (40) and protective cover (42) to be proximally withdrawn through the catheter; and,
wherein the protective cover (42) is a sheath and the sheath inverts to the disengaged position during withdrawal of the internal support (40) and where the sheath has at least one slit (42c) extending along a longitudinal length of the sheath, the at least one slit (42c) for relieving friction between the catheter and the internal support (40).

15. The kit as in claim 14 further comprising the catheter and wherein the internal support (40) and catheter are packaged within a same sterilized packaging.

## Patentansprüche

1. Ein System zum Einführen eines Katheters in ein Gefäßsystem durch eine Gefäßöffnung (VO), das Folgendes beinhaltet:
einen Katheter, der einen inneren Durchmesser und einen äußeren Durchmesser aufweist;
eine innere Stütze (40), die für eine Teleskopbewegung innerhalb des Katheters bemessen ist, wobei die innere Stütze (40) zum Stützen des Katheters und Schützen einer distalen Spitze des Katheters, wenn der Katheter durch eine VO eingeführt wird, vorgesehen ist, wobei die innere Stütze (40) Folgendes aufweist:
eine sich verjüngende distale Spitze (40a) zum Einführen des Systems durch die VO; und
eine Schutzabdeckung (42), die mit der sich verjüngenden distalen Spitze (40a) verbunden ist und sich zum Eingriff über die distale Spitze des Katheters proximal erstreckt, wobei die Schutzabdeckung (42) zwischen einer Eingriffsposition über dem Katheter und einer gelösten Position beweglich ist;
wobei eine selektive Bewegung der inneren Stütze (40) relativ zu dem Katheter bewirkt, dass sich die Schutzabdeckung (42) von der Eingriffsposition in die gelöste Position bewegt, und in der gelösten Position ermöglicht, dass die innere Stütze (40) und die Schutzabdeckung (42) proximal durch den Katheter zurückgezogen werden; und
wobei die Schutzabdeckung (42) eine Hülle ist und sich die Hülle während des Zurückziehens der inneren Stütze (40) in die gelöste Position umstülpt, und
wobei die Hülle mindestens einen Schlitz (42c) aufweist, der sich entlang einer Längslänge der Hülle erstreckt, wobei der Schlitz (42c) zum Mindern von Reibung zwischen dem Katheter und der inneren Stütze (40) vorgesehen ist.

2. System gemäß Anspruch 1, wobei der Katheter einen Katheterendanschlag aufweist und die innere Stütze (40) einen Endanschlag (40b) der inneren Stütze aufweist, und wobei, wenn sich die Schutzabdeckung (40) in der Eingriffsposition befindet, eine Bewegung des Endanschlags (40b) der inneren Stütze in Richtung des Katheterendanschlags bewirkt, dass sich die Schutzabdeckung (42) in die gelöste Position bewegt.

3. System gemäß einem der Ansprüche 1-2, wobei der Katheter ein Aspirationskatheter ist, der einen weichen distalen Spitzenbereich und eine ausreichende Länge aufweist, um sich für eine Behandlung eines ischämischen Schlaganfalls von einer VO einer Speichenarterie zu Hirngefäßen zu erstrecken.

4. System gemäß Anspruch 3, wobei der Aspirationskatheter 5-8F oder 6-8F ist.

5. System gemäß einem der Ansprüche 1-4, wobei die innere Stütze (40) hohl ist und die sich verjüngende distale Spitze (40a) eine Durchgangsbohrung umfasst, die es der inneren Stütze (40) ermöglicht, über einen Draht zu gleiten.

6. System gemäß Anspruch 1, wobei sich der mindestens eine Schlitz (42c) zu einem proximalen Rand der Hülle erstreckt.

7. System gemäß Anspruch 1, wobei der mindestens eine Schlitz (42c) eine Schwächungszone umfasst, die sich während des Umstülpens öffnen kann.

8. System gemäß Anspruch 1, wobei sich der mindestens eine Schlitz (42c) nicht durch einen proximalen Rand der Hülle erstreckt.

9. System gemäß Anspruch 1, wobei der mindestens eine Schlitz (42c) ein Längsschlitz ist.

10. System gemäß Anspruch 1, wobei die Hülle eine oder mehrere Lücken umfasst, um ein Gesamtvolumen der Hülle zu reduzieren und das Umstülpen der Hülle während des Zurückziehens der inneren Stütze zu erleichtern.

11. System gemäß einem der Ansprüche 1-10, das ferner ein Band (60) beinhaltet, das ein proximales Ende der Hülle umgibt, um die Hülle vor der Verwendung auf dem Katheter zu halten.

12. System gemäß Anspruch 1, wobei ein proximaler Rand der Schutzabdeckung (42) ein geformtes Band (43) zum Aufbringen einer Vorspannkraft über den Katheter umfasst, um die Schutzabdeckung (42) in der Eingriffsposition auf dem Katheter zu halten.

13. System gemäß Anspruch 12, wobei das geformte Band (43) ein Thermomaterial ist und wobei die Exposition gegenüber einer Körpertemperatur die Vorspannkraft entspannt.

14. Ein Kit, das Folgendes beinhaltet:
eine innere Stütze (40) innerhalb einer sterilisierten Verpackung, wobei die innere Stütze (40) für eine Teleskopbewegung innerhalb eines Katheters bemessen ist, wobei die innere Stütze (40) zum Stützen des Katheters und Schützen einer distalen Spitze des Katheters, während der Katheter durch eine VO eingeführt wird, vorgesehen ist, wobei die innere Stütze (40) Folgendes aufweist:
eine sich verjüngende distale Spitze (40a) zum Einführen des Kits durch die VO; und
eine Schutzabdeckung (42), die mit der sich verjüngenden distalen Spitze (40a) verbunden ist und sich zum Eingriff über die distale Spitze des Katheters proximal erstreckt, wobei die Schutzabdeckung (42) zwischen einer Eingriffsposition über dem Katheter und einer gelösten Position beweglich ist;
wobei eine selektive Bewegung der inneren Stütze (40) relativ zu dem Katheter bewirkt, dass sich die Schutzabdeckung (42) von der Eingriffsposition in die gelöste Position bewegt, und in der gelösten Position ermöglicht, dass die innere Stütze (40) und die Schutzabdeckung (42) proximal durch den Katheter zurückgezogen werden; und
wobei die Schutzabdeckung (42) eine Hülle ist und sich die Hülle während des Zurückziehens der inneren Stütze (40) in die gelöste Position umstülpt, und wobei die Hülle mindestens einen Schlitz (42c) aufweist, der sich entlang einer Längslänge der Hülle erstreckt, wobei der mindestens eine Schlitz (42c) zum Mindern von Reibung zwischen dem Katheter und der inneren Stütze (40) vorgesehen ist.

15. Kit gemäß Anspruch 14, das ferner den Katheter beinhaltet, und wobei die innere Stütze (40) und der Katheter innerhalb derselben sterilisierten Verpackung verpackt sind.

## Revendications

1. Un système pour introduire un cathéter dans un système vasculaire à travers une ouverture de vaisseau (VO) comprenant :
un cathéter ayant un diamètre interne et un diamètre externe ;
un support interne (40) dimensionné pour un déplacement télescopique à l'intérieur du cathéter, le support interne (40) étant destiné à soutenir le cathéter et protéger une pointe distale du cathéter à mesure que le cathéter est introduit à travers une VO, le support interne (40) ayant :
une pointe distale effilée (40a) destinée à introduire le système à travers la VO ; et,
un capuchon protecteur (42) raccordé à la pointe distale effilée (40a) qui s'étend de manière proximale pour un engagement par-dessus la pointe distale du cathéter, le capuchon protecteur (42) étant mobile entre une position engagée par-dessus le cathéter et une position dégagée ;
dans lequel un déplacement sélectif du support interne (40) par rapport au cathéter amène le capuchon protecteur (42) à se déplacer de la position engagée à la position dégagée et, lorsqu'il est dans la position dégagée, permet au support interne (40) et au capuchon protecteur (42) d'être retirés de manière proximale à travers le cathéter ; et,
dans lequel le capuchon protecteur (42) est une gaine et la gaine se retourne jusqu'à la position dégagée pendant le retrait du support interne (40) et où la gaine a au moins une fente (42c) qui s'étend sur toute une longueur longitudinale de la gaine, la fente (42c) étant destinée à atténuer le frottement entre le cathéter et le support interne (40).

2. Le système tel que dans la revendication 1, dans lequel le cathéter a une butée d'extrémité de cathéter et le support interne (40) a une butée d'extrémité de support interne (40b) et dans lequel, lorsque le capuchon protecteur (40) est dans la position engagée, le déplacement de la butée d'extrémité de support interne (40b) vers la butée d'extrémité de cathéter amène le capuchon protecteur (42) à se déplacer jusqu'à la position dégagée.

3. Le système tel que dans l'une quelconque des revendications 1 à 2 où le cathéter est un cathéter d'aspiration ayant une région de pointe distale souple et une longueur suffisante pour s'étendre d'une VO d'artère radiale jusqu'à des vaisseaux cérébraux pour le traitement d'un accident vasculaire cérébral ischémique.

4. Le système tel que dans la revendication 3 où le cathéter d'aspiration est un cathéter de 5 à 8F ou de 6 à 8F.

5. Le système tel que dans l'une quelconque des revendications 1 à 4 où le support interne (40) est creux, et la pointe distale effilée (40a) inclut un trou traversant permettant au support interne (40) d'avancer sur un fil.

6. Le système tel que dans la revendication 1 où l'au moins une fente (42c) s'étend jusqu'à un bord proximal de la gaine.

7. Le système tel que dans la revendication 1 où l'au moins une fente (42c) inclut une zone de faiblesse capable de s'ouvrir pendant le retournement.

8. Le système tel que dans la revendication 1 où l'au moins une fente (42c) ne s'étend pas d'un côté à l'autre d'un bord proximal de la gaine.

9. Le système tel que dans la revendication 1 où l'au moins une fente (42c) est une encoche longitudinale.

10. Le système tel que dans la revendication 1 où la gaine inclut un ou plusieurs évidements afin de réduire un volume total de la gaine et faciliter le retournement de la gaine pendant le retrait du support interne.

11. Le système tel que dans l'une quelconque des revendications 1 à 10 comprenant en sus une bague (60) entourant une extrémité proximale de la gaine et destinée à maintenir la gaine sur le cathéter avant l'utilisation.

12. Le système tel que dans la revendication 1 dans lequel un bord proximal du capuchon protecteur (42) inclut une bague profilée (43) destinée à appliquer une force de sollicitation sur le cathéter afin de maintenir le capuchon protecteur (42) dans la position engagée sur le cathéter.

13. Le système tel que dans la revendication 12 dans lequel la bague profilée (43) est un matériau thermosensible et dans lequel l'exposition à une température corporelle relâche la force de sollicitation.

14. Un kit comprenant :
un support interne (40) à l'intérieur d'un emballage stérilisé, le support interne (40) étant dimensionné pour un déplacement télescopique à l'intérieur d'un cathéter, le support interne (40) étant destiné à soutenir le cathéter et protéger une pointe distale du cathéter à mesure que le cathéter est introduit à travers une VO, le support interne (40) ayant :
une pointe distale effilée (40a) destinée à introduire le kit à travers la VO ; et,
un capuchon protecteur (42) raccordé à la pointe distale effilée (40a) qui s'étend de manière proximale pour un engagement par-dessus la pointe distale du cathéter, le capuchon protecteur (42) étant mobile entre une position engagée par-dessus le cathéter et une position dégagée ;
dans lequel le déplacement sélectif du support interne (40) par rapport au cathéter amène le capuchon protecteur (42) à se déplacer de la position engagée à la position dégagée et, lorsqu'il est dans la position dégagée, permet au support interne (40) et au capuchon protecteur (42) d'être retirés de manière proximale à travers le cathéter ; et,
dans lequel le capuchon protecteur (42) est une gaine et la gaine se retourne jusqu'à la position dégagée pendant le retrait du support interne (40) et où la gaine a au moins une fente (42c) qui s'étend sur toute une longueur longitudinale de la gaine, l'au moins une fente (42c) étant destinée à atténuer le frottement entre le cathéter et le support interne (40).

15. Le kit tel que dans la revendication 14 comprenant en sus le cathéter et dans lequel le support interne (40) et le cathéter sont emballés à l'intérieur d'un même emballage stérilisé.
